Europäisches Patentamt

European Patent Office

Office européen des brevets

(19)

(11) Numéro de publication: **0 145 558**
**B1**

(12) **FASCICULE DE BREVET EUROPEEN**

(45) Date de publication du fascicule du brevet:
**31.01.90**

(51) Int. Cl.⁴: **A 61 K 9/54, A 61 K 31/40**

(21) Numéro de dépôt: **84402308.5**

(22) Date de dépôt: **13.11.84**

(54) **Nouvelles formes galéniques du sulpiride utilisables par voie orale.**

(30) Priorité: **14.11.83 FR 8318003**

(43) Date de publication de la demande:
**19.06.85 Bulletin 85/25**

(45) Mention de la délivrance du brevet:
**31.01.90 Bulletin 90/5**

(84) Etats contractants désignés:
**AT BE CH DE GB IT LI LU NL SE**

(56) Documents cités:
**EP-A- 0 040 590**
**EP-A- 0 107 557**
**WO-A-82/01649**
**FR-A- 2 453 642**
**FR-A- 2 454 804**

(73) Titulaire: **SOCIETE D'ETUDES SCIENTIFIQUES ET INDUSTRIELLES DE L'ILE-DE-FRANCE, 46, Boulevard de Latour-Maubourg, F-75340 Paris Cédex 07 (FR)**

(72) Inventeur: **Debregeas, Patrice, 10 rue du Peintre Lebrun, F-78000 Versailles (FR)**

ACTORUM AG

## Description

L'invention concerne des nouvelles formes galéniques du Sulpiride, utilisables par voie orale, ainsi que leur procédé de préparation.

Le Sulpiride, de formule développée N-[(éthyl-1 pyrrolidinyl-2)méthyl]méthoxy-2 sulfamoyl-5- benzamide, est connu comme étant un excellent neuroleptique désinhibiteur, ayant comme indication thérapeutique principale le traitement des psychoses aiguës et chroniques, et ses caractéristiques et ses propriétés sont notamment décrites dans les brevets français n° 1 472 025, 4879 M et 5916 M.

Les médicaments actifs par voie orale contenant du Sulpiride se présentent actuellement sous la forme de comprimés, de gélules ou de solutés buvables mais, et les résultats exposés ci-après le démontrent, ces formes actuelles sont relativement mal absorbées par les patients.

On a par conséquent cherché à modifier la forme galénique, de façon à améliorer de façon sensible son acceptation par les patients, en réduisant le nombre de prises journalières, la dose minimale journalière étant de 3 comprimés par jour, dosés à 200 mg de Sulpiride.

La demi-vie plasmatique du principe actif Sulpiride étant d'environ 8 h 30, il apparaît à priori possible de réaliser une forme galénique du produit, permettant de réduire la prise journalière moyenne à 2 gélules ou comprimés par jour, dosés en Sulpiride à 100 mg par gélules ou comprimés (avec une prise le matin et une prise le soir), sinon à 1 gélule ou 1 comprimé par jour, dosé à 200 mg de Sulpiride.

Pour cela, on a réalisé des microgranules à effet prolongé, selon les techniques classiques décrites notamment dans les brevets français n° 2 313 915 (Corneille) ou n° 2 453 642 (Labaz). Classiquement, on a utilisé des grains neutres dont la taille était d'environ 0,50 mm de diamètre, et constitués de 25% d'amidon et de 75% de saccharose, sur lesquels on a projeté en turbine le Sulpiride au moyen d'une solution alcoolique de polyvinylpyrrolidone, puis on a appliqué des fibres d'enrobage classiques, à base de polymères du type gomme laque, méthacrylates ou éthylcellulose, de façon à obtenir une cinétique de libération du principe actif Sulpiride in vitro dans des milieux gastriques et intestinaux pré-établie standard, conformément aux spécifications ci-après:
- 1ère heure: libération inférieure à 40%
- 4ème heure: libération inférieure à 75%
- 8ème heure: libération inférieure à 75%

La libération à la première heure est effectuée dans un suc gastrique artificiel à pH 1,3, à la quatrième heure et à la huitième heure respectivement dans des sucs intestinaux artificiels à pH 4,5 et pH 7. Les conditions opératoires pour étudier la libération du principe actif sont connues et décrites dans les brevets précités.

On a ensuite réalisé avec ces microgranules des gélules et des comprimés dosés à 100 mg et on a étudié l'absorption du Sulpiride en déterminant les taux plasmatiques circulant du Sulpiride par radio-immunologie, puis on a comparé les résultats obtenus avec ceux issus de la forme gélules actuellement sur le marché.

Les résultats sont consignés dans le tableau A ci-après et montrent qu'avec les deux formulations de microgranules utilisés (forme I et forme II), la perte en taux de principe actif circulant est trop importante par rapport à celui de la forme normale en gélules (forme IV), pour permettre de réaliser un médicament à effet prolongé efficace.

A titre indicatif, pour l'homme de l'art, les formes I et II utilisées possédaient les cinétiques suivantes de libération in vitro en milieux artificiels:

|  | forme I | forme II |
|---|---|---|
| Libération à la première heure dans un milieu de pH: 1,3 | 20,0% | 29,2% |
| Libération à la quatrième heure dans un milieu de pH: 4,5 | 68,6% | 58,4% |
| Libération à la huitième heure dans un milieu de pH: 7 | 90,8% | 98,2% |

Après l'étude d'une série de formulations à effet prolongé qui ont toutes donné des résultats incompatibles avec l'obtention d'une bonne forme orale bien absorbée, on a eu l'idée de conserver la forme microgranules (grains sensiblement sphériques de diamètre compris entre 0,2 à 2 mm) qui doit permettre en pratique une meilleure absorption du principe actif qu'une poudre classique de la gélule ou du comprimé, mais en ayant une cinétique de libération in vitro beaucoup plus proche de ces formes immédiates, c'est-à-dire libérant sensiblement 90% du principe actif au bout d'une heure dans un milieu gastrique artificiel de pH 1,3.

De façon surprenante, les résultats des microgranules selon cette cinétique in vitro ont montré que, en fonction des taux plasmatiques comparatifs, cette forme III constituée de microgranules mis en gélules dosées à 100 mg permet d'obtenir une absorption double de celle obtenue avec les gélules classiques de Sulpiride (forme IV) tout en conservant une durée d'action vraisemblable légèrement supérieure.

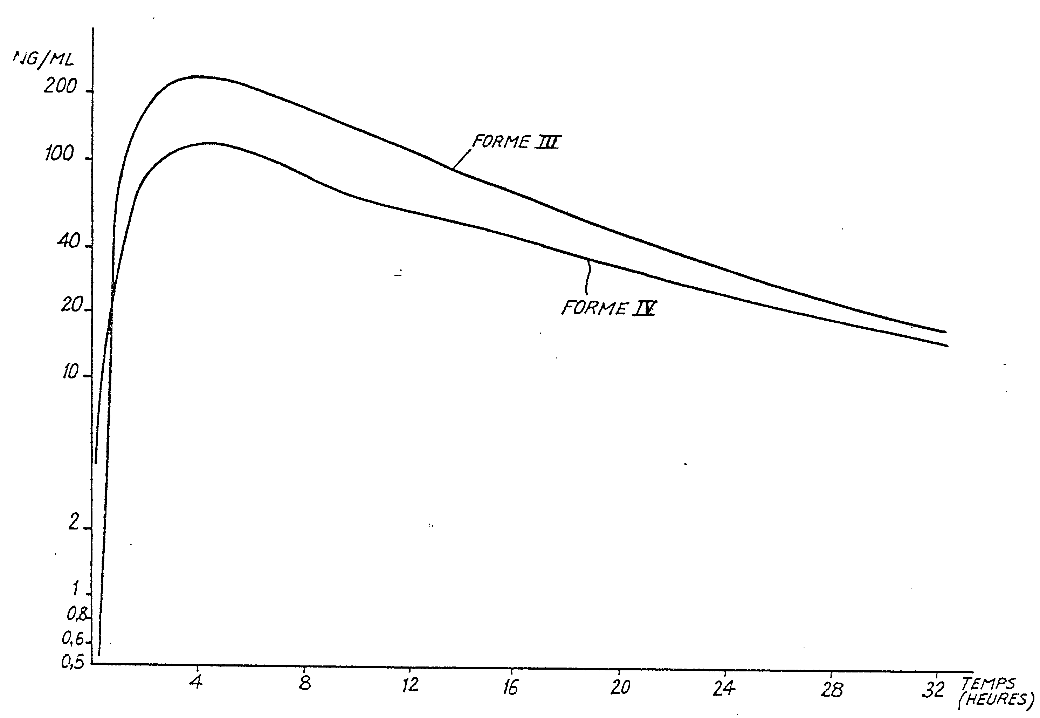

Ces résultats sont exprimés dans le Tableau A, ainsi que par la figure 1, montrant l'évolution des taux plasmatiques circulant exprimés en mg/ml de Sulpiride, entre 0 et 32 heures par comparaison entre la forme III et la forme IV.

Des expérimentations complémentaires au cours desquelles on a fait varier les enrobages externes des microgranules selon l'invention ont permis de déterminer que la forme préférentielle à absorption améliorée du Sulpiride devait répondre sensiblement aux conditions analytiques suivantes de libération in vitro:
15 minutes: libération comprise entre 35 et 50% dans un milieu gastrique artificiel de pH 1,3

30 minutes: libération comprise entre 60 et 75% dans un milieu gastrique artificiel de pH 1,3

60 minutes: libération comprise entre 80 et 95% dans un milieu intestinal artificiel de pH 4,5

Au-delà: libération supérieure à 90% dans un milieu intestinal artificiel de pH 6,9

Comme exemple de réalisation, on a indiqué ci-après la formule de fabrication pour 2500 gélules dosées à 100 mg de la forme III, ainsi que son procédé de fabrication.

On utilise 50 g de grains neutres constitués d'environ 25% d'amidon et de 75% de saccharose, de taille sensiblement égale à 0,50 mm de diamètre, sur lesquels on applique en turbine 250 g de Sulpiride incorporé dans une solution alcoolique (éthanol) de polyvinylpyrrolidone à 20%.

Après séchage et tamisage, on a appliqué des couches extérieures composés de polymères méthacryliques commercialisés sous le nom d'Eudragit (R) par la société Röhm et Haas en solutions alcooliques à raison de 4 parties en poids d'Eudragit type L, pour 1 partie en poids d'Eudragit type RL.

Les Eudragit L sont des copolymères de caractère anionique à base d'acide métacrylique et d'ester méthylique d'acide métacrylique. Ils sont insolubles en milieu acide. Les Eudragit RL sont des copolymérisats de dimétyle amino éthyl métacrylate et d'esters neutres d'acide métacrylique. Ils ont une solubilité indépendante du pH.

On sèche ensuite avec du talc en faibles proportions, jusqu'à l'obtention de microgranules libérant sensiblement 100% en une heure selon les conditions analytiques définies ci-dessus.

On sèche ensuite à l'étuve à 37 °C pendant environ deux jours, puis on termine la couche externe par projection de 40 g d'une solution éthanolique à 13,5% d'Eudragit type RL. Le titre constaté est d'environ 730 mg de principe actif par gramme de microgranules.

Les microgranules ainsi obtenus répondent aux normes de libération in vitro définies ci-dessus, comme étant représentatives des formes galéniques nouvelles du Sulpiride selon l'invention.

Leurs stabilités accélérée et naturelle se sont révélées excellentes sans apparition de toxicité pontentialisée.

Ils peuvent être utilisés en gélules, en comprimés ou en suspension, dans des milieux neutres.

Tableau A

| Paramètres cinétiques | Concentration maximale moyenne (Cmax) en ng/ml | Temps correspondant au Cmax moyen (Tmax) moyen en heure | Aire sous la courbe observée (AUC) | |
|---|---|---|---|---|
| | | | 0–8 heures en ng/ml$^{-1h}$ | 0–24 heures en ng/ml$^{-1h}$ |
| Forme I | 75 ± 40 | 4 ± 1,50 | 340 ± 75 | 720 ± 190 |
| Forme II | 80 ± 45 | 3,90 ± 1,40 | 365 ± 80 | 780 ± 200 |
| Forme III | 315 ± 65 | 3,30 ± 1,30 | 1480 ± 230 | 2970 ± 400 |
| Forme IV | 150 ± 60 | 3,20 ± 1,30 | 715 ± 320 | 1565 ± 430 |

**Revendications pour les Etats contractants: BE, CH, DE, GB, IT, LI, LU, NL, SE**

1. Nouvelle forme galénique du Sulpiride, utilisable par voie orale, comprenant des microgranules constitués d'un grain neutre formé d'un mélange de saccharose et d'amidon, représentant environ 20% de la composition, d'une couche intermédiaire constituée de polyvinylpyrrolidone et de sulpiride, représentant environ 70% de la composition et d'une enveloppe extérieure formée d'une première couche constituée d'un mélange de polymères méthacryliques comportant environ 4 parties en poids d'Eudragit L pour 1 partie en poids d'Eudragit RL et d'une deuxième couche constituée d'Eudragit RL.

2. Procédé de préparation d'une forme galénique selon la revendication 1, caractérisé en ce que l'on projette en turbine, sur des grains neutres, une solution alcoolique de poylvinylpyrrolidone contenant du sulpiride, que l'on sèche et tamise les microgranules obtenus, que l'on applique une solution alcoolique du mélange de polymères Eudragit L et Eudragit RL, que l'on sèche avec du talc puis à l'étuve à 37 °C et enfin, que l'on termine l'enveloppe extérieure en projetant une solution alcoolique de polymère Eudragit RL.

**Revendication pour l'Etat contractant: AT**

Procédé de préparation d'une nouvelle forme galénique du Sulpiride utilisable par voie orale, caractérisé en ce que l'on projette en turbine, sur des grains neutres constitués d'un mélange de saccharose et d'amidon et représentant environ 20% de la composition, une solution alcoolique de polyvinylpyrrolidone contenant du Sulpiride, représentant environ 70% de la composition, que l'on sèche et tamise les microgranules ainsi obtenus, que l'on applique une solution alcoolique d'un mélange de polymères méthacryliques comportant environ 4 parties en poids de polymère Eudragit L pour 1 partie en poids de polymère Eudragit RL, que l'on sèche avec du talc puis à l'étuve à 37 °C et enfin que l'on termine la couche extérieure en projetant une solution alcoolique de polymère Eudragit RL.

## Claims for the Contracting States BE, CH, DE, GB, IT, LI, LU, NL, SE

1. Novel galenic form of sulpiride, which can be used orally, comprising microgranules constituted by a neutral grain formed by a mixture of saccharose and starch, representing about 20% of the composition, an intermediate layer constituted by polyvinyl-pyrrolidone and sulpiride, representing about 70% of the composition, and an outer casing formed by a first layer constituted by a mixture of methacrylic polymers comprising about 4 parts by weight of Eudragit L for 1 part by weight of Eudragit RL, and a second layer of Eudragit RL.

2. A process for preparing a galenic form according to claim 1 characterised in that an alcohol solution of poylvinylpyrrolidone containing sulpiride is projected in a turbine process on to neutral grains, that the microgranules obtained are dried and sifted, that an alcohol solution of the mixture of polymers Eudragit L and Eudragit RL is applied, that drying is effected with talc, then in a drying oven at 37 °C, and finally the outer casing is completed by projecting an alcohol solution of polymer Eudragit RL.

## Claim for the Contracting State AT

A process for preparing a novel galenic form of sulpiride which can be used orally, characterized in that an alcohol solution of poylvinylpyrrolidone containing sulpiride, representing about 70% of the composition, is projected in a turbine process on to neutral grains constituted by a mixture of saccharose and starch, representing about 20% of the composition, that the resulting microgranules are dried and sifted, that an alcohol solution of methacrylic polymers containing about 4 parts by weight of polymer Eudragit L for 1 part by weight of polymer Eudragit RL is applied, that drying is effected with talc then in a drying oven at 37 °C and finally that the outer layer is completed by projecting an alcohol solution of polymer Eudragit RL.

## Patentansprüche für die Vertragsstaaten BE, CH, DE, GB, IT, LI, LU, NL, SE

1. Neue galenische Form des Sulpirids zur Verwendung auf oralem Wege, enthaltend Mikrokapseln, bestehend aus einem Gemisch von Saccharose und Stärke gebildeten, neutralen Korn, das etwa 20% der Zusammensetzung ausmacht, einer aus Polyvinylpyrrolidon und Sulpirid bestehenden Zwischenschicht, die etwa 70% der Zusammensetzung ausmacht, und einer äußeren Umhüllung, die aus einer ersten, aus einem Gemisch von Methacrylpolymeren, enthaltend etwa 4 Gewichtsteile Eudragit L auf einen Gewichtsteil Eudragit RL, bestehenden Schicht und einer zweiten, aus Eudragit RL bestehenden Schicht gebildet ist.

2. Verfahren zur Herstellung einer galenischen Form nach Anspruch 1, dadurch gekennzeichnet, daß man in einem Turbinenprozeß auf neutrale Körner eine alkoholische, Sulpirid enthaltende Lösung von Polyvinylpyrrolidon aufträgt bzw. aufsprüht, daß man die erhaltenen Mikrokapseln trocknet und siebt, daß man eine alkoholische Lösung des Gemisches der Polymeren Eudragit L und Eudragit RL aufträgt, daß man mit Talcum und anschließend im Trockenofen bei 37 °C trocknet, und daß man die äußere Umhüllung durch Auftragen bzw. Aufsprühen einer alkoholischen Lösung des Polymeren Eudragit RL fertigstellt.

## Patentanspruch für den Vertragsstaat AT

Verfahren zur Herstellung einer neuen galenischen Form des Sulpirids zur Verwendung auf oralem Wege, dadurch gekennzeichnet, daß man in einem Turbinenprozeß auf neutrale Körner, die aus einem Gemisch von Saccharose und Stärke bestehen und etwa 20% der Zusammensetzung ausmachen, eine alkoholische, Sulpirid enthaltende Lösung von Polyvinylpyrrolidon, die etwa 70% der Zusammensetzung ausmacht, aufträgt bzw. aufsprüht, daß man die so erhaltenen Mikrokapseln trocknet und siebt, daß man eine alkoholische Lösung eines Gemisches von Methacrylpolymeren, enthaltend etwa 4 Gewichtsteile Polymeren Eudragit L auf ein Gewichtsteil des Polymeren Eudragit RL aufträgt, daß man mit Talcum und anschließend im Trockenofen bei 37 °C trocknet, und daß man schließlich die äußere Umhüllung durch Auftragen bzw. Aufsprühen einer alkoholischen Lösung des Polymeren Eudragit RL fertigstellt.